(19) Europäisches Patentamt
European Patent Office
Office européen des brevets

(11) Veröffentlichungsnummer: **0 008 340**
**B1**

(12) **EUROPÄISCHE PATENTSCHRIFT**

(45) Veröffentlichungstag der Patentschrift: **21.12.83**

(21) Anmeldenummer: **79102223.9**

(22) Anmeldetag: **02.07.79**

(51) Int. Cl.³: **C 07 C 69/743,**
A 01 N 53/00,
C 07 C 49/16,
C 07 C 49/527,
C 07 C 51/09,
C 07 C 51/245,
C 07 C 51/58,
C 07 C 61/16,
C 07 C 67/08,
C 07 C 67/14, C 07 C 69/63

(54) **Fluoralkenylsubstituierte Cyclopropancarbonsäureester, Zwischenprodukte dafür, Herstellung der Verbindungen und ihre Verwendung als Insektizide und/oder Akarizide.**

(30) Priorität: **15.07.78 DE 2831193**

(43) Veröffentlichungstag der Anmeldung:
**05.03.80 Patentblatt 80/5**

(45) Bekanntmachung des Hinweises auf die Patenterteilung
**21.12.83 Patentblatt 83/51**

(84) Benannte Vertragsstaaten:
**AT BE CH DE FR GB IT NL SE**

(56) Entgegenhaltungen:
**EP - A - 0 003 336**
**DE - A - 2 802 962**
**DE - A - 2 822 472**

**Chem. Soc. Reviews, 1978, Vol. 7, Heft 4, S. 473-505**

**Die Akte enthält technische Angaben, die nach dem Eingang der Anmeldung eingereicht wurden und die nicht in dieser Patentschrift enthalten sind.**

(73) Patentinhaber: **BAYER AG**
**Zentralbereich Patente, Marken und Lizenzen**
**D-5090 Leverkusen 1, Bayerwerk (DE)**

(72) Erfinder: **Lantzsch, Reinhard, Dr.**
**Heymannstrasse 32**
**D-5090 Leverkusen 1 (DE)**
Erfinder: **Hagemann, Hermann, Dr.**
**Roggendorfstrasse 55**
**D-5000 Köln 80 (DE)**
Erfinder: **Hammann, Ingeborg, Dr.**
**Belfortstrasse 9**
**D-5000 Köln 1 (DE)**
Erfinder: **Behrenz, Wolfgang, Dr.**
**Untergründemich 14**
**D-5063 Overath-Steinenbrück (DE)**
Erfinder: **Homeyer, Bernhard, Dr.**
**Obere Strasse 18**
**D-5090 Leverkusen 3 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

EP 0 008 340 B1

Fluoralkenylsubstituierte Cyclopropancarbonsäureester, Zwischenprodukte dafür, Herstellung der Verbindungen und ihre Verwendung als Insektizide und/oder Akarizide

Die vorliegende Erfindung betrifft neue fluoralkenylsubstituierte Cyclopropancarbonsäureester, Verfahren zu ihrer Herstellung, ihre Verwendung als Insektizide sowie neue Zwischenprodukte zur Herstellung dieser Wirkstoffe.

Chloralkenylsubstituierte Cyclopropancarbonsäureester sind bereits bekannt aus der deutschen Offenlegungsschrift 23 26 077.

Alkenylsubstituierte Cyclopropancarbonsäureester sind bekannt aus der deutschen Patentschrift 19 26 433.

Diese daraus bekannten Verbindungen besitzen jedoch den Nachteil zu geringer Wirksamkeit. Vor allem bei niedrigen Aufwandkonzentrationen ist Wirkung und Wirkungsspektrum nicht befriedigend.

1. Es wurden die neuen fluor alkenylsubstituierten Cyclopropancarbonsäureester der Formel I gefunden

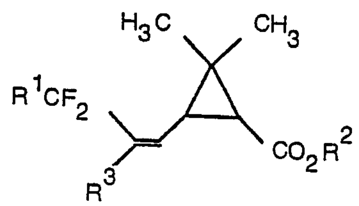

(I)

in welcher

$R^1$ für Fluor oder $CF_3$ steht,

$R^3$ für Fluor, Chlor oder Brom oder den Rest $R^1CF_2$-stehen und

$R^2$ für den Rest der Formel steht:

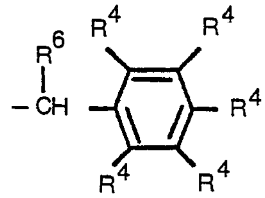

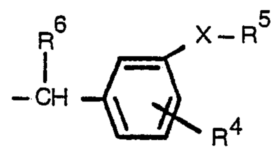

wobei

X für $—CH_2—$, —O—, oder —S— steht,

$R^4$ für Wasserstoff, Halogen oder $CF_3$ steht,

$R^5$ für $—CH_2—C{\equiv}CH$, $—CHF_2$, $—CF_3$, $—CH{=}CCl_2$ oder einen gegebenenfalls durch ein oder mehrere Halogenatome, Methyl- oder Methoxygruppen substituierten Phenylrest steht,

$R^6$ für H, oder CN steht,

ausgenommen die Verbindungen 2,2-Dimethyl-3-(2′-chlor-3′,3′,3′-trifluorpropenyl)-cyclopropancarbonsäure-3′-phenoxybenzylester und -3′-phenoxy-$\alpha$-CN-benzylester.

Dieser und die in die Ansprüche 5 und 6 aufgenommenen disclaimer betreffen die Vertragsstaaten DE, BE, CH, FR, GB, NL, SE und gehen auf die EP 0 003 336 zurück.

2. Es wurde ferner gefunden, daß man die neuen fluoralkenylsubstituierten Cyclopropancarbonsäureester der Formel I erhält, indem man eine Säure oder deren reaktionsfähiges Derivat der Formel II

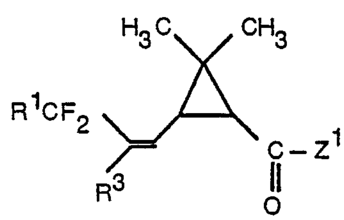

(II)

in welcher

$R^1$ und $R^3$ die unter 1. angegebene Bedeutung haben und

$Z^1$ Halogen, vorzugsweise Chlor, oder OH bedeutet, mit einem Alkohol oder dessen reaktionsfähigem Derivat der Formel III

$$R^2—Z^2 \qquad (III)$$

in welcher

$R^2$ die unter 1. angegebene Bedeutung hat und

$Z^2$ OH, Cl oder Br bedeutet,

gegebenenfalls in Gegenwart von Lösungsmitteln, Säureakzeptoren und/oder Phasentransferkatalysatoren umsetzt.

3. Es wurden ferner die neuen Säuren, bzw. die reaktionsfähigen Derivate derselben, der Formel II gefunden,

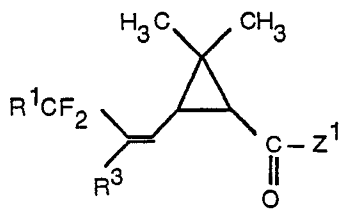

(II)

in welcher

$R^1$, $R^3$ und $Z^1$ die unter 2. angegebene Bedeutung besitzen.

4. Es wurde ferner gefunden, daß man die neuen Säuren, bzw. die reaktionsfähigen Derivate derselben, der Formel II erhält, indem man die Verbindungen der Formel IV

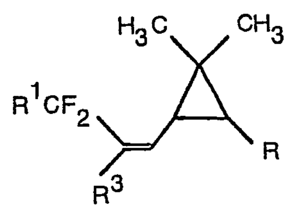

(IV)

in welcher

R für $—COOC_{1-4}$-Alkyl, CN oder $—COCH_3$ steht und $R^1$ und $R^3$ die oben angegebene Bedeutung besitzen für den Fall, daß R für

$$—\overset{O}{\overset{\|}{C}}O—C_{1-4}\text{-Alkyl}$$

oder CN steht verseift oder für den Fall, daß R für

$$—\overset{O}{\overset{\|}{C}}—CH_3$$

steht, oxidiert und gegebenenfalls die Säure, in welcher R COOH bedeutet mit einem Halogenierungsmittel umsetzt.

5. Es wurden ferner die neuen Verbindungen der Formel IV gefunden,

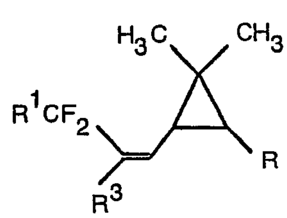

(IV)

in welcher

$R^1$ und $R^3$ die unter 1. angegebene Bedeutung besitzen und R für CN,

$$—\overset{O}{\overset{\|}{C}}—CH_3 \text{ oder } —\overset{O}{\overset{\|}{C}}O—C_{1-4}\text{-Alkyl steht.}$$

6. Es wurde ferner gefunden, daß man die neuen Verbindungen der Formel IV erhält, indem man die Verbindungen der Formel V

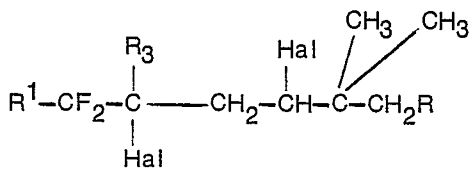

(V)

in welcher
$R^1$, $R^3$ und R die oben angegebene Bedeutung besitzen, und
Hal für Chlor oder Brom steht dehydrohalogeniert.

7. Verbindungen der Formel V

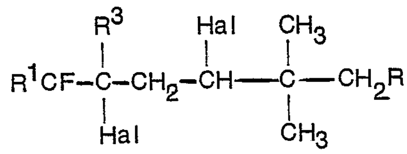

(V)

in welcher
$R^1$, $R^3$ und Hal die unter 6. und
R die unter 5. angegebene Bedeutung besitzen,
werden erhalten, indem man die Halogenverbindungen der Formel VI

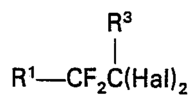

(VI)

in welcher
$R^1$ Fluor oder $CF_3$ und
Hal gleich oder verschieden ist und Chlor oder Brom bedeutet und $R^3$ die unter 1. angegebene Bedeutung besitzt,
an Olefine der Formel VII

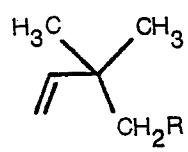

(VII)

in welcher
R die unter 5. angegebene Bedeutung besitzt, gegebenenfalls in Gegenwart eines Katalysators, addiert.

Die Verbindungen der Formel I gemäß 1. zeigen gute insektizide Eigenschaften.

Überraschenderweise zeigen die neuen erfindungsgemäßen Wirkstoffe gemäß 1. eine erheblich höhere Wirksamkeit als die aus dem Stand der Technik bekannten Verbindungen.

Von den erfindungsgemäßen Verbindungen der Formel I gemäß 1. sind diejenigen bevorzugt, in denen $R^1$ für Fluor oder $CF_3$ steht, $R^3$, Chlor oder Brom bedeutet und $R^2$.

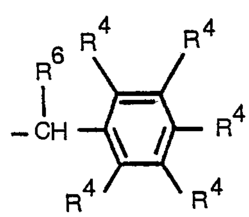

oder

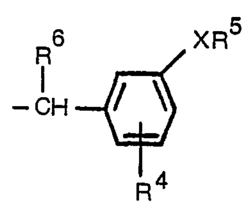

bedeutet,
wobei

X für $—CH_2—$, —O— oder —S— steht
$R^6$ für Wasserstoff, oder CN steht
$R^4$ für Wasserstoff, Halogen oder $CF_3$ steht
$R^5$ für $—CH_2—C{\equiv}CH$, $—CHF_2$, $—CF_3$, $—CH{=}CCl_2$ oder einen gegebenenfalls durch Halogen, Methyl oder Methoxy substituierten Phenylrest steht.

Besonders bevorzugt sind Verbindungen in denen $R^2$ für

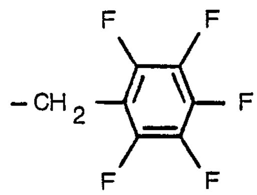

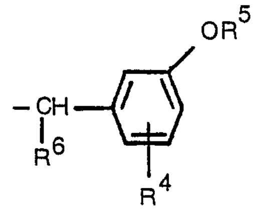

steht, worin

$R^6$ für H, CN steht

$R^5$ für $—CH_2C≡CH$, $—CHF_2$, $—CF_3$, $—CH{=}CCl_2$ oder einen gegebenenfalls durch ein oder mehrere Halogenatome, Methylgruppen oder Methoxygruppen substituiertes Phenyl steht, und

$R^4$ für H oder Halogen steht.

Ganz besonders bevorzugt sind solche Verbindungen der Formel I il welcher $R^1$ für Fluor steht, Hal Chlor oder Brom bedeutet und $R^2$ für

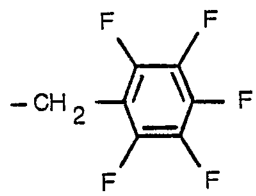

oder

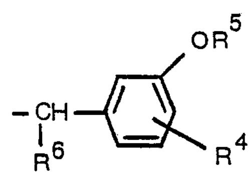

steht, wobei $R^6$ für

H oder CN steht, $R^4$ für Wasserstoff oder Halogen insbesondere Fluor und

$R^5$ für gegebenenfalls durch ein oder mehrere Halogenatome, Methyl- oder Methoxygruppen substituiertes Phenyl steht

Folgende Verbindungen der Formel I seien im einzelnen gennant:

Pentafluorbenzyl-2,2-dimethyl-3-(2′-chlor-3′,3′,3′-trifluorpropenyl)-cyclopropancarboxylat,

Pentafluorbenzyl-2,2-dimethyl-3-(2′-brom-3′,3′,3′-trifluorpropenyl)-cyclopropancarboxylat,

3′-Phenoxybenzyl-2,2-dimethyl-3-(2′-chlor-3′,3′,3′-trifluor-propenyl)-cyclopropancarboxylat,

3′-Phenoxy-$\alpha$′-cyanobenzyl-2,2-dimethyl-3(2′-chlor-3′,3′,3′-trifluor-propenyl)-cyclopropancarboxylat,

3′-Phenoxybenzyl-2,2-dimethyl-3-3(2′-brom-3′,3′,3′-trifluor-propenyl)-cyclopropancarboxylat,

3′-Phenoxy-$\alpha$′-cyanobenzyl-2,2-dimethyl-3-(2′-brom-3′,3′,3′-trifluor-propenyl)-cyclopropancarboxylat,

3′-Phenoxy-$\alpha$′-cyanobenzyl-2,2-dimethyl-3-(2′-chlor-3′,3′,4′,4′,4′-pentenfluor-butenyl)-cyclopropancarboxylat,

3′-Phenoxybenzyl-2,2-dimethyl-3-(2′-fluor-3′,3′,3′-trifluorpropenyl)-cyclopropancarboxylat,

3′-Phenoxy-$\alpha$′-cyanobenzyl-2,2-dimethyl-3-(2′-fluor-3′,3′,3′-trifluorpropenyl)-cyclopropancarboxylat,

3′-Phenoxybenzyl-2,2-dimethyl-3-(2′-trifluormethyl-3′,3′,3′-trifluorpropenyl)-cyclopropancarboxylat,

3′-Phenoxy-$\alpha$′-cyanobenzyl-2,2-dimethyl-3-(2′-trifluormethyl-3′,3′,3′-trifluorpropenyl)-cyclopropancarboxylat,

3′-Propargyloxybenzyl-2,2-dimethyl-3-(2′-chlor-3′,3′,3′-trifluor-propenyl)-cyclopropancarboxylat,

3′-Dichlorvinyloxybenzyl-2,2-dimethyl-3-(2′-chlor-3′,3′,3′-trifluor-propenyl)-cyclopropancarboxylat,

3′-Difluormethoxybenzyl-2,2-dimethyl-3-(2′-chlor-3′,3′,3′-trifluor-propenyl)-cyclopropancarboxylat,

3′-Phenoxy-4′-fluor-$\alpha$′-cyanobenzyl-2,2-dimethyl-3-(2′-chlor-3′,3′,3′-trifluor-propenyl)-cyclopropancarboxylat,

3′-Phenoxy-4-′-fluor-benzyl-2,2-dimethyl-3-(2′-brom-3′,3′,3′-trifluor-propenyl)-cyclopropancarboxylat,

3′-(4′-Fluorphenoxy)-benzyl-2,2-dimethyl-3-(2′-chlor-3′,3′,3′-trifluor-propenyl)-cyclopropancarboxylat,

3′-(4′-Chlorphenoxy)-benzyl-2,2-dimethyl-3-(2′-chlor-3′,3′,3′-trifluor-propenyl)-cyclopropancarboxylat,

3′-(4′-Bromphenoxy)-$\alpha$′-cyanobenzyl-2,2-dimethyl-3-(2′-chlor-3′,3′,3′-trifluor-propenyl)-cyclopropancarboxylat.

Die Herstellung der erfindungsgemäßen fluoralkenylsubstituierten Cyclopropancarbonsäureester kann durch folgendes Reaktionsschema wiedergegeben werden:

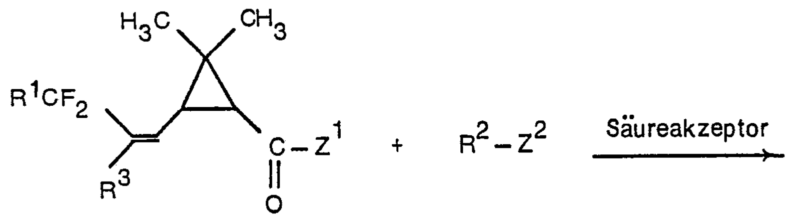

I

Verwendet man beispielsweise gemäß Verfahren 2. 2,2-Dimethyl-3-(2'-brom-3',3',3'-trifluor-propenyl)-cyclopropancarbonsäurechlorid und 3-Phenoxybenzylalkohol als Ausgangsmaterialien, so kann der Reaktionsverlauf durch das folgende Formelschema wiedergegeben werden:

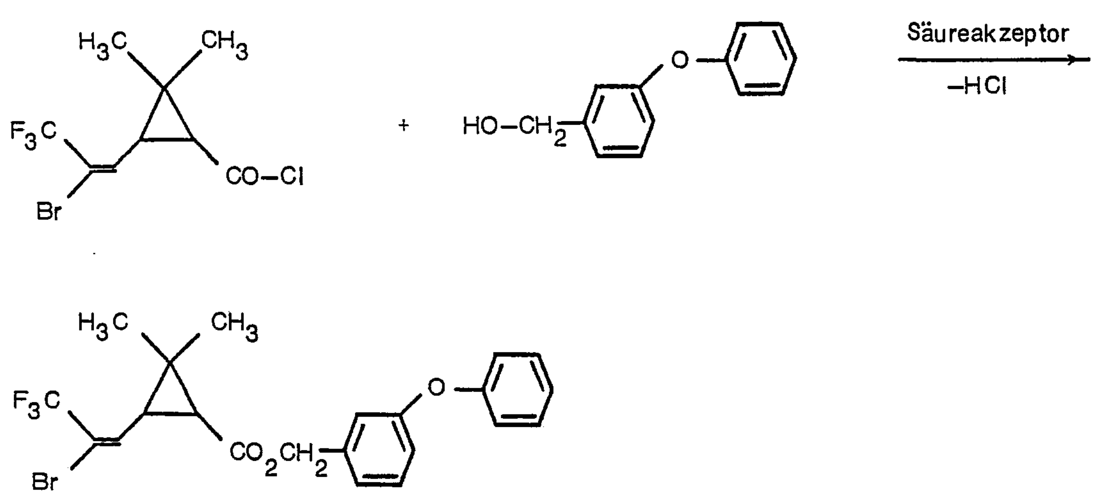


Die als Ausgangsprodukte zu verwendenden Cyclopropancarbonsäuren bzw. deren reaktionsfähigen Derivate der Formel II sind neu. Als reaktionsfähige Derivate bevorzugt die Säurechloride eingesetzt.

Als Beispiele für die als Ausgangsprodukte zu verwendenden Verbindungen der Formel II seien im einzelnen genannt:

2,2-Dimethyl-3-(2'-chlor-3',3',3'-trifluor-propenyl)-cyclopropancarbonsäure (und -chlorid)
2,2-Dimethyl-3-(2'-brom-3',3',3'-trifluor-propenyl)-cyclopropancarbonsäure (und -chlorid)
2,2-Dimethyl-3-(2'-chlor-3',3',4',4',4'-pentanfluorbutenyl)-cyclopropancarbonsäure (und -chlorid)
2,2-Dimethyl-3-(2'-brom-3',3',4',4',4'-pentafluorbutenyl)-cyclopropancarbonsäure (und -chlorid).
2,2-Dimethyl-3-(2'-fluor-3',3',3'-trifluorpropenyl)-cyclopropancarbonsäure (und -chlorid),
2,2-Dimethyl-3-(2'-trifluormethyl-3',3',3'-trifluorpropenyl)-cyclopropancarbonsäure (und -chlorid).

Die neuen Verbindungen der Formel II können nach dem unter 4. angegebenen Verfahren hergestellt werden (Einzelheiten siehe weiter unten).

Die ebenfalls als Ausgangsprodukte zu verwendenden Alkohols bzw. reaktionsfähigen Derivate der Formel III sind bekannt und nach allgemein üblichen, in der Literatur beschriebenen Verfahren herstellbar (vgl. z.B. DT—AS oder DT—OS 25 54 883, 19 26 433, 26 12 115, 19 26 433, 24 36 178, 24 36 462 sowie Monatshefte 67, Seite 35 (1936)).

Bevorzugt sind Verbindungen der Formel III in welcher $R^2$ die angebene bevorzugte oder besonders bevorzugte Bedeutung hat.

Als Beispiele für die als Ausgangsprodukte zu verwendenden Verbindungen der Formel III seien im einzelnen genannt:

Pentafluorbenzylalkohol
3-Trifluormethoxybenzylalkohol
3-Dichlorvinyloxybenzylalkohol
3-Propargyloxybenzylalkohol
3-Dichlorvinyloxy-α-cyano-benzylalkohol
3-Phenoxy-benzylalkohol
3-Phenyl-α-cyano-benzylalkohol
3-Phenoxy-4-fluor-benzylalkohol
3-Phenoxy-4-chlor-benzylalkohol

3-Phenoxy-4-fluor-$\alpha$-cyanobenzylalkohol
3-(4'-Fluorphenoxy)-benzylalkohol
3-(4'-Chlorphenoxy)-benzylalkohol
3-(4'-Bromphenoxy)-benzylalkohol
3-Difluormethoxy-benzylchlorid
3-Phenoxy-benzylbromid
Pentafluorbenzylchlorid

Zur Herstellung der erfindungsgemäßen Verbindungen der Formel I gemäß 1. aus Carbonsäuren bzw. Carbonsäurehalogeniden der Formel II und Alkoholen bzw. Chloriden oder Bromiden der Formel III können als Säureakzeptoren alle üblichen Säurebindemittel Verwendung finden.

Besonders bewährt haben sich Alkalihydroxide, -carbonate und -alkoholate, wie Kaliumhydroxid, Natriumhydroxid, Natriummethylat, Kaliumcarbonat, Natriumäthylat, ferner aliphatische, aromatische oder heterocyclische Amine, beispielsweise Triäthylamin, Trimethylamin, Dimethylanilin, Dimethylbenzylamin und Pyridin.

Die Reaktionstemperatur kann innerhalb eines größeren Bereiches variiert werden. Im allgemeinen arbeitet man bei der Umsetzung der Säurehalogenide mit Alkoholen zwischen 0 und 100°C, vorzugsweise bei 15 bis 40°C und bei der Umsetzung der Carbonsäure mit den Halogeniden zwischen 50 und 150°C, vorzugsweise bei 80°C bis 120°C. Im letzteren Falle wird bevorzugt in Gegenwart eines Katalysators gearbeitet.

Als Katalysatoren kommen alle sogenannten Phasentransferkatalysatoren in Betracht, wie beispielsweise Kronenäther oder quartäre Ammonium- oder Phosphoniumsalze. Bevorzugt sind quartäre Ammoniumsalze, wie beispielsweise Tetrabutylammoniumchlorid, Tetrabutylammoniumbromid, Benzyltriäthylammoniumchlorid oder Methyltrioctylammoniumchlorid.

Die Umsetzung läßt man im allgemeinen bei Normaldruck ablaufen. Das Verfahren zur Herstellung der erfindungsgemäßen Verbindungen wird bevorzugt unter Mitverwendung geeigneter Lösung- und Verdünnungsmittel durchgeführt. Als solche kommen praktisch alle inerten organischen Solventien in Frage. Hierzu gehören insbesondere aliphatische und aromatische, gegebenenfalls chlorierte Kohlenwasserstoffe wie Benzol, Toluol, Xylol, Benzin, Methylenchlorid, Chloroform, Dichloräthan, Chlorbenzol, o-Dichlorbenzol oder Äther, z.B. Diäthyl-, Diisopropyl oder Dibutyläther, außerdem Nitrile, wie Aceto- und Propionitril.

Zur Durchführung des Verfahrens setzt man die Ausgangskomponenten vorzugsweise in äquimolaren Verhältnissen ein. Die Reaktionskomponenten werden im allgemeinen in einem der angegebenen Lösungsmittel zusammengegeben und meist bei erhöhter Temperatur nach Zugabe des Säureakzeptors und gegebenenfalls des Katalysators zur Vervollständigung der Reaktion eine oder mehrere Stunden gerührt. Anschließend gießt man das Reaktionsgemisch in Wasser, trennt die organische Phase ab und wäscht diese mit Wasser neutral. Nach dem Trocknen wird das Lösungsmittel im Vakuum abdestilliert. Die neuen Verbindungen fallen in Form von Ölen an, die sich zum Teil nicht unzersetzt destillieren lassen, jedoch durch sogenanntes "Andestillieren", d.h. durch längeres Erhitzen unter vermindertem Druck auf mäßig erhöhte Temperaturen von den letzten flüchtigen Anteilen befreit und auf diese Weise gereinigt werden. Zu ihrer Charakterisierung dient der Brechnungsindex.

Die neuen Säuren bzw. Säurehalogenide der Formel II lassen sich nach dem unter 4. (oben)angegebenen Verfahren aus dem Verbindungen der Formel IV herstellen.

Bei Variante 4 a wird für den Fall, daß R für

$$-\overset{\overset{\displaystyle O}{\|}}{C}O-C_{1-4}\text{-Alkyl}$$

steht in an sich üblicher Weise sauer oder alkalisch verseift. Bevorzugt ist die alkalische Verseifung.

Für den Fall, daß R für CN steht, wird die Nitrilgruppe in eine Estergruppe umgewandelt, indem man das Nitril in dem entsprechenden Alkohol $C_{1-4}$-Alkyl-OH löst und die Lösung mit Chlorwasserstoff sättigt, üblicherweise bei etwa 0—20°C. Nach einigen Stehen bildet sich dabei das Imidchlorid, aus dem mit Wasser der Ester entsteht.

Für die Verseifung kommen alle üblichen Verseifungsmittel in Frage, wie beispielsweise Schwefelsäure, Essigsäure oder Alkalilaugen. Besonders bevorzugt ist KOH in alkoholischer Lösung, z.B. in Methanol.

Die Verseifung kann bei erhöhter Temperatur durchgeführt werden, beispielsweise zwischen 20 und 100°C, bevorzugt wird jedoch bei 20°—40°C gearbeitet.

Die Säuren werden durch Ansäuern mit wäßrigen Säuren in Freiheit gesetzt und durch Extraktion von den Salzen abgetrennt, sowie gegebenenfalls durch Destillation gereinigt.

Die Säurehalogenide können nach den üblichen Methoden mit den üblichen Halogenierungsmitteln, wie beispielsweise $SOCl_2$, $COCl_2$, $PCl_3$, $PCl_5$, Oxalylchlorid oder -bromid, $PBr_3$, hergestellt werden.

Bei Variante 4 b steht R für

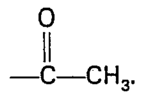

Man erhält die Säure durch Oxidation, vorzugsweise mit Hypochlorit (Chlorlauge) oder Hypobromit. Beispiele für solche Haloformreagentien sind Natriumhypochlorit, Kaliumhypochlorit, Calziumhypochlorit, Natriumhypobromit, Kaliumhypobromit und Calziumhypobromit. Ferner kann die Reaktion auch durchgeführt werden, indem man ein Halogen in eine die Ausgangsverbindung enthaltende wäßrige Alkalihydroxidlösung einleitet. Die Reaktionstemperatur beträgt im allgemeinen —20° bis 100°C, vorzugsweise 0—70°C. Im allgemeinen wird Wasser als Lösungsmittel verwendet, es können aber auch organische Lösungsmittel als Lösungsvermittler zugesetzt werden. Durch Zusatz von Phasentransferkatalysatoren, wie beispielsweise quartären Ammoniumsalzen, kann eine Beschleunigung der Reaktion erreicht werden. Als quartäre Ammoniumsalze kommen die oben bereits genannten in Frage.

Als Beispiele für die neuen Verbindungen der Formel IV seien im einzelnen genannt:
2,2-Dimethyl-3-(2′-chlor-3′,3′,3′-trifluorpropenyl)-cyclopropancarbonsäuremethylester,
2,2-Dimethyl-3-(2′-chlor-3′,3′,3′-trifluorpropenyl)-cyclopropancarbonsäureäthylester,
2,2-Dimethyl-3-(2′-chlor-3′,3′,3′-trifluorpropenyl)-cyclopropancarbonsäurebutylester,
2,2-Dimethyl-3-(2′-brom-3′,3′,3′-trifluor-propenyl)-cyclopropancarbonsäuremethylester,
1-Cyano-2,2-dimethyl-3-(2′-chlor-3′,3′,3′-trifluorpropenyl)-cyclopropan,
1-Cyano-2,2-dimethyl-3-(2′-chlor-3′,3′,3′-trifluorpropenyl)-cyclopropan,
1-Acetyl-2,2-dimethyl-3-(2′-chlor-3′,3′,3′-trifluorpropenyl)-cyclopropan,
2,2-Dimethyl-3-(2′-trifluormethyl-3′,3′,3′-trifluorpropenyl)-cyclopropancarbonsäureäthylester.

Die neuen Verbindungen der Formel IV lassen sich nach dem unter 6. angegebenen Verfahren aus den Verbindungen der Formel V durch Dehydrohalogenierung herstellen. Diese Reaktion läßt sich durch folgendes Formelschema darstellen:

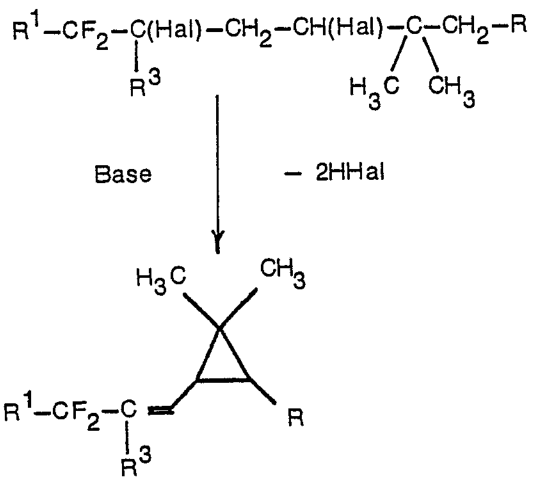


Die Dehydrohalogenierung erfolgt durch Basen in Anwesenheit von Verdünnungsmitteln. Als Basen kommen dabei in Frage:

NaOH, KOH, $K_2CO_3$, Alkoholate, wie Natriummethylat, Natriumisopropylat oder Kalium-tert.-Butylat. Als Lösungsmittel kommen in erster Linie Alkohole in Frage, wie beispielsweise Methanol, Äthanol, n- oder iso-Propanol, Butanol, tert.-Butanol, Glykol oder Glykol-monomethyläther; ebenso aber auch gegebenenfalls chlorierte Kohlenwasserstoffe oder Äther, wie Benzol, Toluol, Xylol, Chlorbenzol, Dichlorbenzol, Methylenchlorid, THF, Dioxan, Dimethoxyäthan. Es kann auch, insbesondere bei Verwendung von NaOH oder KOH, in einem Zweiphasensystem — wie beispielsweise Toluol/Wasser gearbeitet werden. Gegebenenfalls kann ein Phasentransferkatalysator, wie beispielsweise ein quartäres Ammoniumsalz, zugefügt werden. Dies ist nicht nur beim Arbeiten im Zweiphasensystem sinnvoll, sondern auch beim wasserfreien Arbeiten von Vorteil, wenn beispielsweise Kaliumcarbonat als Base Verwendung findet.

Eine ähnliche Reaktion ist in der DT—OS 25 39 896 beschrieben. Doch während dort der bevorzugte Temperaturbereich — bei Anwendung von Natriummethylat oder Natriumäthylat — zwischen 60 und 100°C liegt (Seite 31), wird unter diesen Bedingungen nur sehr wenig Produkt der Formel IV erhalten, sondern hauptsächlich ein weiteres Mol Halogenwasserstoff abgespalten unter Bildung eines Produkts der Formel VIII

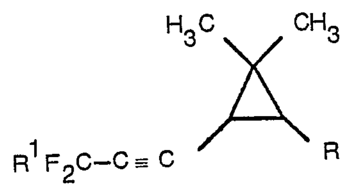

VIII

wobei R und $R^1$ die angegebene Bedeutung besitzen.

Überraschend wurde gefunden, daß praktisch kein Produkt der Formel VIII gebildet wird, wenn man die Base, vorzugsweise $NaOCH_3$ oder $NaOC_2H_5$, in einem Lösungsmittel vorlegt, dann die zu dehydrohalogenierende Verbindung der Formel I langsam zufügt und zwar zunächst bei etwa 0—30°C und dan bis zur Beendigung der Reaktion bei 20—60°C, vorzugsweise bei 35—50°C. Hierbei ist besonders überraschend, daß die Dehydrohalogenierung bereits bei diesen niedrigen Temperaturen abläuft.

Als Beispiele für Verbindungen der Formel V seien genannt:

3,3-Dimethyl-4,6,6-trichlor-7,7,7-trifluor-önanthsäuremethylester
3,3-Dimethyl-4,6,6-trichlor-7,7,7-trifluor-önanthsäureäthylester
3,3-Dimethyl-4,6,6-trichlor-7,7,7-trifluor-önanthsäurebutylester
3,3-Dimethyl-4,6,6-trichlor-7,7,7-trifluor-önanthsäurenitril,
3,3-Dimethyl-4,6,6-tribrom-7,7,7-trifluor-önanthsäuremethylester
2,2-Dimethyl-3,5,5-trichlor-6,6,6-trifluor-hexylmethylketon,
3,3-Dimethyl-4,6-dichlor-6-trifluormethyl-7,7,7-trifluor-önanthsäureäthylester.

Die neuen Verbindungen der Formel V werden nach dem Verfahren 7. (oben) aus den Verbindungen der Formel VI und VII erhalten. Die Verbindungen der Formel VI und VII sind bekannt.

Das Verfahren wird durch folgendes Reaktionsschema wiedergegeben:

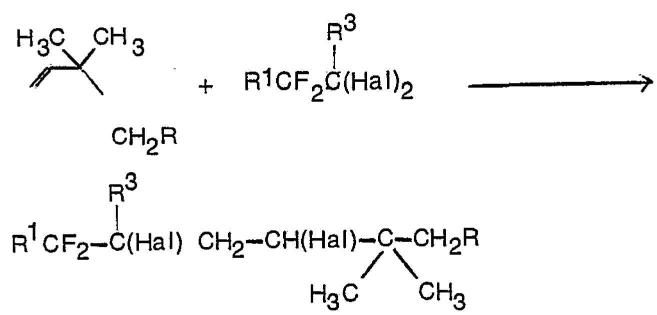

Verwendet man beispielsweise den 3,3-Dimethyl-4-pentensäure-methylester und 1,1,1-Trichlor-2,2,2-trifluor-äthan als Ausgangsstoffe, so läßt sich der Reaktionsverlauf durch folgendes Formelschema darstellen:

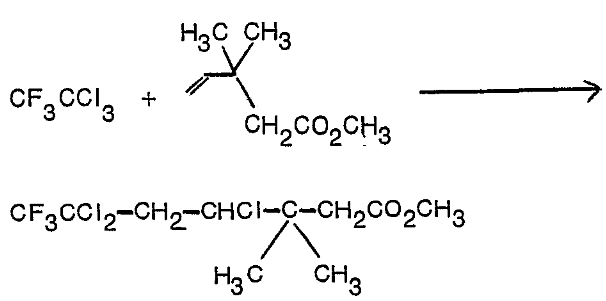

Folgende Ausgangsstoffe der Formel VI seien beispielsweise genannt:

1,1,1-Trichlor-2,2,2-trifluoräthan
1,1,1-Tribrom-2,2,2-trifluoräthan
1-Chlor-1,1-dibrom-2,2,2-trifluoräthan
1,1-Dichlor-1-brom-2,2,2-trifluoräthan
1,1,1-Trichlor-2,2,3,3,3-pentafluorpropan
1,1,1-Tribrom-2,2,3,3,3-pentafluorpropan
1,1,1,2-Tetrafluor-2,2-dichloräthan,
1,1,1,3,3,3-Hexafluor-2,2-dichlorpropan,
1,1,1,2-Tetrafluor-2,2-dibromäthan,
1,1,1,3,3,3-Hexafluor-2,2-dibrompropan.

Folgende Ausgangsstoffe der Formel VII seien beispielsweise genannt:
3,3-Dimethyl-4-pentensäuremethylester
3,3-Dimethyl-4-pentensäureäthylester
3,3-Dimethyl-4-pentensäurebutylester
3,3-Dimethyl-4-pentensäurenitril
2,2-Dimethyl-3-butenyl-methylketon.

Das Verfahren wird vorzugsweise unter Druck, kann jedoch auch bei Verwendung von höhersiedenden Verbindungen der Formel VI bei Normaldruck durchgeführt werden.

Der Druckbereich kann in weiten Grenzen schwanken, etwa zwischen 1 und 30 bar, bevorzugt zwischen 3 und 15 bar. Der Überdruck kann durch Aufpressen eines Inertgases, beispielsweise von Stickstoff, erreicht werden.

Die Umsetzungstemperatur kann zwischen 50 und 200°C liegen, bevorzugt wird jedoch zwischen 100°C und 150°C gearbeitet. Den beiden Ausgangsstoffen wird gegebenenfalls ein Verdünnungsmittel zugesetzt.

Als Verdünnungsmittel kommen in Frage: aliphatische oder aromatische Kohlenwasserstoffe, wie Benzin oder Toluol, Alkohole, wie Methanol Äthanol, Propanol oder tert. Butanol, Nitrole wie Acetonitril oder Propionitril, DMF. Bevorzugt sind Alkohole und Acetonitril.

Zur Durchführung der Reaktion sind Katalysatoren erforderlich. Und zwar entweder

a) freie Radikale liefernde Katalysatoren, wie z.B. Azobisisobutyronitril, Benzylperoxid oder Di-tert.-butylperoxid.

Die Verbindungen der Formel VI werden äquimolar oder aber bevorzugt im Überschuß eingesetzt. Oder

b) Katalysatormischungen, die aus einem Metallsalz, einem Amin und gegebenenfalls etwas Benzoin bestehen. Das Metallsalz kann auch als Hydrat vorliegen, das Amin kann auch als Salz, z.B. als Hydrochlorid vorliegen.

Beispiele für Metallsalze sind:

Kupfer(I)chlorid, Kupfer(II)chlorid, Eisen(II)-chlorid, Eisen(III)-chlorid, Kupfer(II)-sulfat, Kobaltchlorid, Zinkchlorid, Eisensulfat, Eisenacetat, Kupfer- oder Eisenacetylacetonat.

Beispiele für Amine sind:

Dimethylamin, Diäthylamin, Trimethylamin, Triäthylamin, Diisopropylamin, n-Butylamin, Benzylamin, Äthanolamin, Anilin, Pyridin. Bevorzugt sind Dimethylamin, Diäthylamin (als Hydrochloride), sowie n-Butylamin.

Es werden vorzugsweise mindestens 1,5 Mol, bevorzugt 2—10 Mol organischen Amin pro Mol Metallsalz eingesetzt. Das Metallsalz wird in Mengen von 0,01 bis 15 Mol-%, vorzugsweise 0,05 bis 10 Mol-% bezogen auf Verbindungen der Formel VII eingesetzt.

Die Zugabe von äquimolaren Mengen Benzoin bezogen auf das Metallsalz ist oft von Vorteil.

Die Wirkstoffe eignen sich bei guter Pflanzenverträglichkeit und günstiger Warmblütertoxizität zur Bekämpfung von tierischen Schädlingen, insbesondere Insekten und Spinnentieren, die in der Landwirtschaft, in Forsten, im Vorrats- und Materialschutz sowie auf dem Hyginesektor vorkommen. Sie sind gegen normal sensible und resistente Arten sowie gegen alle oder einzelne Entwicklungsstadien wirksam. Zu den oben erwähnten Schädlingen gehören:

Aus der Ordnung der Isopoda z.B. Oniscus asellus, Armadillidium vulgare, Porcellio scaber.

Aus der Ordnung der Diplopoda z.B. Blaniulus guttulatus.

Aus der Ordnung der Chilopoda z.B. Geophilus carpophaus, Scutigera spec.

Aus der Ordnung der Symphyla z.B. Scutigerella immaculata.

Aus der Ordnung der Thysanura z.B. Lepisma saccharina.

Aus der Ordnung der Collembola z.B. Onychiurus armatus.

Aus der Ordnung der Orthoptera z.B. Blatta orientalis, Periplaneta americana, Leucophaea maderae, Blattella germanica, Acheta domesticus, Gryllotalpa spp., Locusta migratoria migratorioides, Melanoplus differentialis, Schistocerca gregaria.

Aus der Ordnung der Dermaptera z.B. Forficula auricularia.

Aus der Ordnung der Isoptera z.B. Reticulitermes spp.

Aus der Ordnung der Anoplura z.B. Phylloxera vastatrix, Pemphigus spp., Pediculus humanus corporis, Haematopinus spp., Linognathus spp.

Aus der Ordnung der Mallophaga z.B. Trichodectes spp., Damalinea spp.

Aus der Ordnung der Thysanoptera z.B. Hercinothrips fermoralis, Thrips tabaci.

Aus der Ordnung der Heteroptera z.B. Eurygaster spp., Dysdercus intermedius, Piesma quadrata, Cimex lectularius, Rhodnius prolixus, Triatoma spp.

Aus der Ordnung der Homoptera z.B. Aleurodes brassicae, Bemisia tabaci, Trialeurodes vaporariorum, Aphis gossypii, Brevicoryne brassicae, Cryptomyzus ribis, Doralis fabae, Doralis pomi, Eriosoma lanigerum, Hyalopterus arundinis, Macrosiphum avenae, Myzus spp., Phorodon humuli, Rhopalosiphum padi, Empoasca spp., Euscelis bilobatus, Nephotettix cincticeps, Lecanium corni, Saissetia oleae, Laodelphax striatellus, Nilaparvata lugens, Aonidiella aurantii, Aspidiotus hederae, Pseudococcus spp. Psylla spp.

Aus der Ordnung der Lepidoptera z.B. Pectinophora gossypiella, Bupalus piniarius, Cheimatobia

brumata, Lithocolletis blancardella, Hyponomeuta padella, Plutella maculipennis, Malacosoma neustria, Euproctis chrysorrhoea, Lymantria spp. Bucculatrix thurberiella, Phyllocnistis citrella, Agrotis spp., Euxoa spp., Feltia spp. Earias insulana, Heliothis spp., Laphygma exigua, Mamestra brassicae, Panolis flammea, Prodenia litura, Spodoptera spp., Trichoplusia ni, Carpocapsa pomonella, Pieris spp., Chilo spp., Pyrausta nubilalis, Ephestia kuehniella, Galleria mellonella, Cacoecia podana, Capua reticulana, Choristoneura fumiferana, Clysia ambiguella, Homona magnanima, Tortrix viridana.

Aus der Ordnung der Coleoptera z.B. Anobium punctatum, Rhizopertha dominica, Bruchidius obtectus, Acanthoscelides obtectus, Hylotrupes bajulus, Agelastica alni, Leptinotarsa decemlineata, Phaedon cochleariae, Diabrotica spp., Psylliodes chrysocephala, Epilachna varivestis, Atomaria spp., Oryzaephilus surinamensis, Anthonomus spp., Sitophilus spp., Otiorrhynchus sulcatus, Cosmopolites sordidus, Ceuthorrhynchus assimilis, Hypera postica, Dermestes spp., Trogoderma spp., Anthrenus spp., Attagenus spp., Lyctus spp., Meligethes aeneus, Ptinus spp., Niptus hololeucus, Gibbium psylloides, Tribolium spp., Tenebrio molitor, Agriotes spp., Conoderus spp., Melolontha melolontha, Amphimallon solstitialis, Costelytra zealandica.

Aus der Ordnung der Hymenoptera z.B. Diprion spp., Hoplocampa spp., Lasius spp., Monomorium pharaonis, Vespa spp.

Aus der Ordnung der Diptera z.B. Aedes spp., Anopheles spp., Culex spp., Drosophila melanogaster, Musca spp., Fannia spp., Calliphora erythrocephala, Lucilia spp., Chrysomyia spp., Cuterebra spp., Gastrophilus spp., Hyppobosca spp., Stromoxys spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae, Tipula paludosa.

Aus der Ordnung der Siphonaptera z.B. Xenopsylla cheopis, Ceratophyllus spp.

Aus der Ordnung der Arachnida z.B. Scorpio maurus, Latrodectus mactans.

Aus der Ordnung der Acarina z.B. Acarus siro, Argas spp., Ornithodoros spp. Dermanyssus gallinae, Eriophyes ribis, Phyllocoptruta oleivora, Boophilus spp., Rhipicephalus spp., Amblyomma spp., Hyalomma spp., Ixodes spp., Psoroptes spp., Chorioptes spp., Sarcoptes spp., Tarsonemus spp., Bryobia praetiosa, Panonychus spp., Tetranychus spp.

Die Wirkstoffe können in die üblichen Formulierungen übergeführt werden, wie Lösungen, Emulsionen, Suspensionen, Pulver, Schäume, Pasten, Granulate, Aerosole, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä., sowie ULV-Kalt- und Warmnebel-Formulierungen.

Diese Formulierungen werden in au sich bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chloräthylene oder Methylenchlorid, aliphatische Kohlenwasserstoffe, wie Cyclohexan oder Paraffine, z.B. Erdölfraktionen, Alkohole, wie Butanol oder Glycol sowie deren Äther und Ester, Ketone, wie Aceton, Methyläthylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgas, wie Halogenkohlenwasserstoffe sowie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier- und/oder schaumerzeugende Mittel kommen in Frage: z.B. nichtionogene und anionische Emulgatoren, wie Polyoxyäthylen-Fettsäure-Ester, Polyoxyäthylen-Fettalkohol-Äther, z.B. Alkylarylpolyglykol-äther, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat.

Es können Farbstoffe wie anorganische Pigmente, z.B. Eisenoxid, Titanoxid, Ferrocyanblau und organsiche Farbstoffe, wie Alizarin-, Azol-Metallphthalocyaninfarbstoffe und Spurenährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90%.

Die Anwendung der erfindungsgemäßen Wirkstoffe erfolgt in Form ihrer handelsüblichen Formulierungen und/oder den aus diesen Formulierungen bereiteten Anwendungsformen.

Der Wirkstoffgehalt der aus den handelsüblichen Formulierungen bereiteten Anwendungsformen kann in weiten Bereichen variieren. Die Wirkstoffkonzentration der Anwendungsformen kann von 0,0000001 bis zu 100 Gew.-% Wirkstoff, vorzugsweise zwischen 0,01 und 10 Gew.-% liegen.

Die Anwendung geschieht in einer den Anwendungsformen angepaßten üblichen Weise.

Bei der Anwendung gegen Hygiene- und Vorratsschädlinge zeichnen sich die Wirkstoffe durch eine hervorragende Residualwirkung auf Holz und Ton sowie durch eine gute Alkalistabilität auf gekälkten Unterlagen aus.

Die Anwendung der erfindungsgemäßen Wirkstoffe geschieht im Veterinärsektor in au sich bekannter Weise, wie durch orale Anwendung in Form von beispielsweise Tabletten, Kapseln, Tränken, Granulaten, durch dermale Anwendung in Form beispielsweise des Tauchens (Dippen), Sprühens (Sprayen), Aufgießens (pour-on and spot-on) und des Einpuderns sowie durch parenterale Anwendung in Form beispielsweise der Injektion.

Beispiel A
Phaedon-Larven-Test

Lösungsmittel: 3 Gewichtsteile Dimethylformamid

Emulgator: 1 Gewichtsteil Alkylarylpolyglykoläther

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Kohlblätter (Brassica oleracea) werden durch Tauchen in die Wirkstoffzubereitung der gewünschter. Konzentration behandelt und mit Meerrettichblattkäfer-larven (Phaedon cochleariae) besetzt, solange die Blätter noch feucht sind.

Nach der gewünschten Zeit wird die Abtötung in % bestimmt. Dabei bedeutet 100%, daß alle Käfer-Larven abgetötet wurden; 0% bedeutet, daß keine Käfer-Larven abgetötet wurden.

Bei diesem Test zeigen z.B. die folgenden Verbindungen der Herstellungsbeispiele überlegene Wirksamkeit gegenüber dem Stand der Technik: 2, 4.

Beispiel B
Tetranychus-Test (resistent)

Lösungsmittel: 3 Gewichtsteile Dimethylformamid

Emulgator: 1 Gewichtsteil Alkylarylpolyglykoläther

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Bohnenpflanzen (Phaseolus vulgaris), die stark von allen Entwicklungsstadien der gemeinen Spinnmilbe oder Bohnenspinnmilbe (Tetranychus urticae) befallen sind, werden durch Tauchen in die Wirkstoffzubereitung der gewünschten Konzentration behandelt.

Nach der gewünschten Zeit wird die Abtötung in % bestimmt. Dabei bedeutet 100%, daß alle Spinnmilben abgetötet wurden; 0% bedeutet, daß keine Spinnmilben abgetötet wurden.

Bei diesem Test zeigen z.B. die folgenden Verbindungen der Herstellungsbeispiele überlegene Wirksamkeit gegenüber dem Stand der Technik: 2, 4.

Beispiel C
Grenzkonzentrations-Test/Bodeninsekten

| | | |
|---|---|---|
| Testinsekt: | Tenebrio Molitor | (im Boden) |
| Lösungsmittel: | 3 Gewichtsteile | Aceton |
| Emulgator: | 1 Gewichtsteil | Alkylarylpolyglykoläther |

Zur Herstellung einer zweckmäßigen Wirkstoffzubereitung vermischt man 1 Gewichtsteil Wirkstoff mit der angegebenen Menge Lösungsmittel gibt die angegebene Menge Emulgator zu und verdünnt das Konzentrat mit Wasser auf die gewünschte Konzentration.

Die Wirkstoffzubereitung wird innig mit dem Boden vermischt. Dabei spielt die Konzentration des Wirkstoffs in der Zubereitung praktisch keine Rolle, entscheidend ist allein die Wirkstoffgewichtsmenge pro Volumeneinheit Boden, welche in ppm (= mg/l) angegeben wird. Man füllt den Boden in Töpfe und läßt diese bei Raumtemperatur stehen.

Nach 24 Stunden werden die Testtiere in den behandelten Boden gegeben und nach weiteren 2 bis 7 Tagen wird der Wirkungsgrad des Wirkstoffs durch Auszählen der toten und lebenden Testinsekten in % bestimmt. Der Wirkungsgrad ist 100%, wenn alle Testinsekten abgetötet worden sind, er ist 0%, wenn noch genau so viele Testinsekten leben wie bei der unbehandelten Kontrolle.

Bei diesem Test zeigen z.B. die folgenden Verbindungen der Herstellungsbeispiele überlegene Wirkung gegenüber dem Stand der Technik: 1, 3.

Beispiel D

$LD_{100}$-Test

Testtiere: Blatta orientalis

Zahl der Testtiere: 10

Lösungsmittel: Aceton

2 Gewichtsteile Wirkstoff werden in 1000 Volumenteilen Lösungsmittel aufgenommen. Die so erhaltene Lösung wird mit weiterem Lösungsmittel auf die gewünschten Konzentrationen verdünnt.

2,5 ml Wirkstofflösung werden in eine Petrischale pipettiert. Auf dem Boden der Petrischale befindet sich ein Filterpapier mit einem Durchmesser von etwa 9,5 cm. Die Petrischale bleibt so lange offen stehen, bis das Lösungsmittel vollständig verdunstet ist. Je nach Konzentration der Wirkstofflösung ist die Menge Wirkstoff pro $m^2$ Filterpapier verschieden hoch. Anschließend gibt man die angegebene Anzahl der Testtiere in die Petrischale und bedeckt sie mit einem Glasdeckel.

Der Zustand der Testtiere wird 3 Tage nach Ansetzen der Versuche kontrolliert. Bestimmt wird die Abtötung in %. Dabei bedeutet 100%, daß alle Testtiere abgetötet wurden; 0% bedeutet, daß keine Testtiere abgetötet wurden.

Bei diesem Test zeigen z.B. die folgenden Verbindungen der Herstellungsbeispiele überlegene Wirkung gegenüber dem Stand der Technik: 1, 2, 4.

Beispiel E

$LT_{100}$-Test für Dipteren

Testtiere: Aedes aegypti

Zahl der Testtiere: 25

Lösungsmittel: Aceton

2 Gewichtsteile Wirkstoff werden in 1000 Volumenteilen Lösungsmittel aufgenommen. Die so erhaltene Lösung wird mit weiterem Lösungsmittel auf die gewünschten geringeren Konzentrationen verdünnt.

2,5 ml Wirkstofflösung werden in eine Petrischale pipettiert. Auf dem Boden der Petrischale befindet sich ein Filterpapier mit einem Durchmesser von etwa 9,5 cm. Die Petrischale bleibt so lange offen stehen, bis das Lösungsmittel vollständig verdunstet ist. Je nach Konzentration der Wirkstofflösung ist die Menge Wirkstoff pro $m^2$ Filterpapier verschieden hoch. Anschließend gibt man die angegebene Anzahl der Testtiere in die Petrischale und bedeckt sie mit einem Glasdeckel.

Der Zustand der Testtiere wird laufend kontrolliert. Es wird diejenige Zeit ermittelt, welche für einen 100%igen knock down-Effekt notwendig ist.

Bei diesem Test zeigen z.B. die folgenden Verbindungen der Herstellungsbeispiele überlegene Wirkung gegenüber dem Stand der Technik: 1, 2, 3, 4.

Beispiel 1

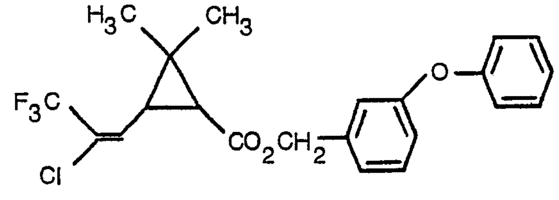

5 g trans-2,2-Dimethyl-3-(2'-chlor-3',3',3'-trifluorpropenyl)-cyclopropancarbonsäurechlorid werden mit 100 ml Toluol und 3,85 g 3-Phenoxybenzylalkohol vermischt. Dann tropft man bei Raumtemperatur unter Rühren 1,5 g Pyridin in 50 ml Toluol zu und rührt 4 h bei Raumtemperatur nach. Dann wird das Reaktionsgemisch mit 150 ml Wasser versetzt, die organische Phase abgetrennt und mit 100 ml Wasser gewaschen. Die organische Phase wird mit Natriumsulfat getrocknet, abfiltriert, einrotiert und bei 60°C im Hochvakuum andestilliert (ca. 1 h).

Der Rückstand wiegt 6,1 g und besteht aus trans-2,2-Dimethyl-3-(2'-chlor-3',3',3'-trifluorpropenyl)-cyclopropancarbonsäure-(3'-phenoxy)-benzylester. $n_D^{20} = 1.5292$.

Beispiel 2

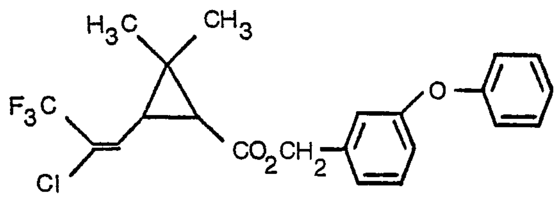

Analog Beispiel 1 erhält man aus 3,2 g cis-2,2-Dimethyl-3-(2'-chlor-3',3',3'-trifluorpropenyl)-cyclopropancarbonsäurechlorid, 2,44 g 3-Phenoxybenzylalkohol und 0,97 g Pyridin 3,2 g cis-2,2-Dimethyl-3-(2'-chlor-3',3',3'-trifluorpropenyl)-cyclopropancarbonsäure-(3'-phenoxy)-benzylester. $n_D^{20} = 1,5295$.

Beispiel 3

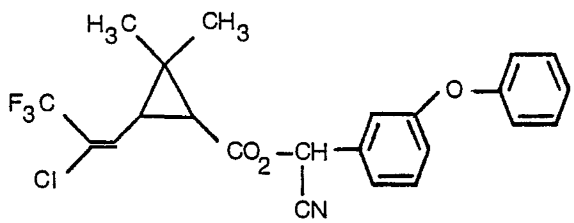

5 g trans-2,2-Dimethyl-3-(2'-chlor-3',3',3'-trifluorpropenyl)-cyclopropancarbonsäurechlorid werden mit 100 ml Toluol und 4,28 g Cyanhydrin des 3-Phenoxybenzaldehydes vermischt. Dann tropft man bei Raumtemperatur unter Rühren 1,5 g Pyridin in 50 ml Toluol zu und rührt 4 h bei Raumtemperatur nach. Dann wird das Reaktionsgemisch mit 150 ml Wasser versetzt, die organische Phase abgetrennt und mit 100 ml Wasser gewaschen. Die organische Phase wird mit Natriumsulfat getrocknet, abfiltriert, einrotiert und bei 60°C im Hochvakuum andestilliert (ca. 1 h). Der Rückstand wiegt 7,2 g und besteht aus trans-2,2-Dimethyl-3-(2'-chlor-3',3',3'-trifluorpropenyl)-cyclopropancarbonsäure-(3'-phenoxy)-$\alpha$-cyanobenzylester. $n_D^{20}$: 1.5281.

Beispiel 4

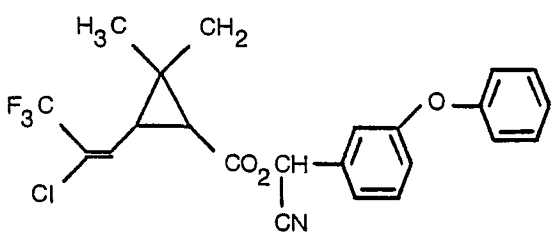

Analog Beispiel 3 erhält man aus 3,2 g cis-2,2-Dimethyl-3-(2'-chlor-3',3',3'-trifluorpropenyl)-cyclopropancarbonsäurechlorid, 2,75 g Cyanhydrin des 3-Phenoxybenzaldehyds und 0,97 g Pyridin, 4,4 g cis-2,2-Dimethyl-3-(2'-chlor-3',3',3'-trifluorpropenyl)-cyclopropancarbonsäure-(3'-phenoxy)-$\alpha$-cyano-benzylester. $n_D^{20}$: 1.5271.

Analog den Beispielen 1—4 werden folgende Verbindungen hergestellt, wobei Isomeren-Gemische des Säurechlorids als Ausgangsstoffe verwendet werden:

| Beispiel | Verbindung (Formel) |
|---|---|
| 5 | 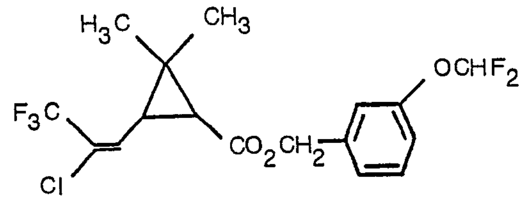 |
| 6 | 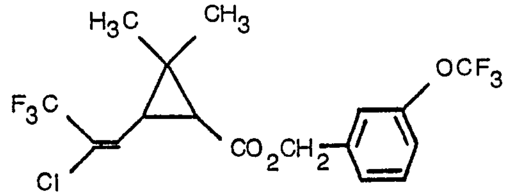 |
| 7 | 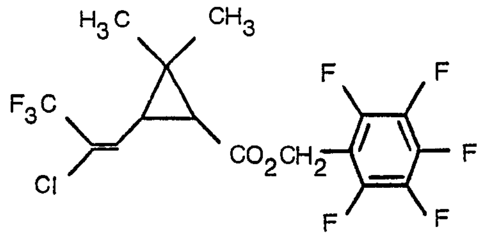 |
| 8 | 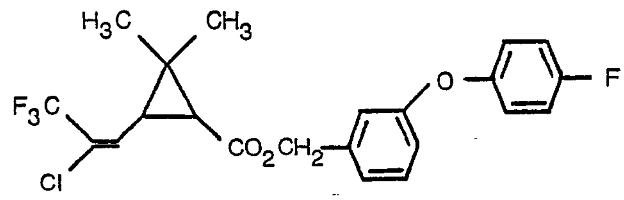 |
| 9 | 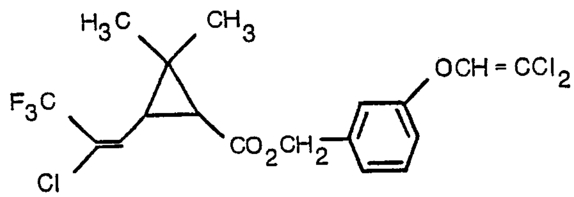 |
| 10 | 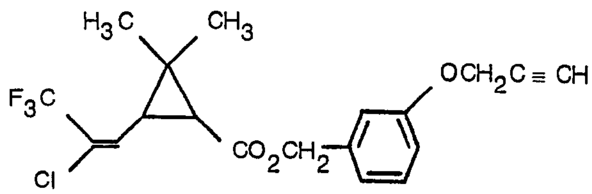 |
| 11 | 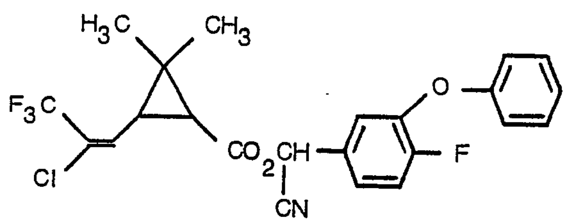 |
| 12 | 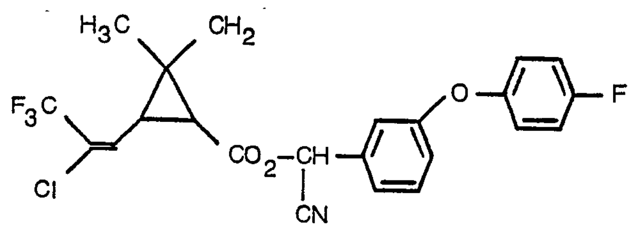 |

Beispiel 13

Herstellung von

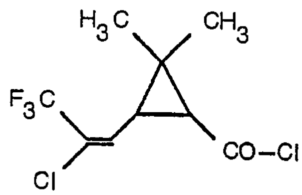

Man löst 20 g trans-2,2-Dimethyl-3-(2′-chlor-3′,3′,3′-trifluorpropenyl)-cyclopropancarbonsäure in 150 ml Methylenchlorid, gibt einige Tropfen DMF hinzu und leitet bei Raumtemperatur solange Phosgen ein, bis die Reaktion beendet ist. (Kontrolle durch das Infrarotspektrum). Anschließend wird überschüssiges Phosgen und Methylenchlorid abdestilliert. Der Rückstand destilliert bei 40—50°C/0,5 mm (0,665 mbar). Der Brechungsindex des trans-2,2-Dimethyl-3-(2′-chlor-3′,3′,3′-trifluorpropenyl)-cyclopropancarbonsäurechlorids beträgt $n_D^{20}$: 1.4554.

Beispiel 14

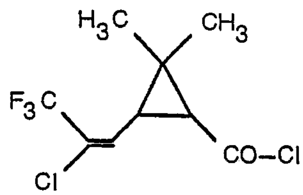

Man löst 10 g cis-2,2-Dimethyl-3-(2′-chlor-3′,3′,3′-trifluorpropenyl)-cyclopropancarbonsäure in 50 ml Thionylchlorid und erhitzt eine Stunde zum Sieden. Nach Abdestillieren des überschüssigen Thionylchlorids wird der Rückstand im Hochvakuum destilliert. Man erhält das cis-2,2-Dimethyl-3-(2′-chlor-3′,3′,3′-trifluorpropenyl)-cyclopropancarbonsäurechlorid vom Kp. = 52—54°C/0,9 mm (1,197 mbar) und $n_D^{20}$: 1.4520.

Beispiel 15

Auf die gleiche Weise wie in Beispiel 13 oder 14 erhält man das 2,2-Dimethyl-3-(2′-chlor-3′,3′,3′-trifluorpropenyl)-cyclopropancarbonsäurechlorid aus 2,2-Dimethyl-3-(2′-chlor-3′,3′,3′-trifluorpropenyl)-cyclopropancarbonsäure, wobei ein Stereoisomerengemisch eingesetzt wurde, das alle 4 möglichen Stereoisomeren enthielt.

Beispiel 16

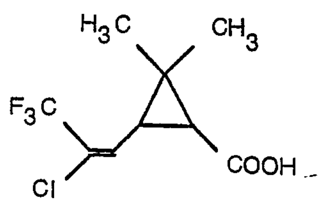

18 g trans-2,2-Dimethyl-3-(2′-chlor-3′,3′,3′-trifluorpropenyl)-cyclopropancarbonsäureäthylester werden mit 100 ml 3 N methanolischer KOH versetzt und 6 h bei Raumtemperatur gerührt. Anschließend versetzt man mit 300 ml Eiswasser und stellt mit konz. HCl auf pH 1. Durch Extrahieren mit Petroläther erhält man 11,2 g Carbonsäure vom Smp. 110—111°C.

Beispiel 17

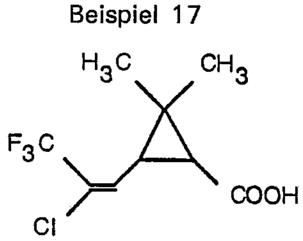

Analog Beispiel 16 werden aus 15 g cis-2,2-Dimethyl-3-(2′-chlor-3′,3′,3′-trifluorpropenyl)-cyclopropancarbonsäureäthylester 9,4 g cis-Carbonsäure vom Smp. 90—91°C erhalten.

Beispiel 18

Analog Beispiel 16 erhält man aus 2,2-Dimethyl-3-(2′-chlor-3′,3′,3′-trifluorpropenyl)-cyclopropancarbonsäureäthylester (Isomerengemisch) die 2,2-Dimethyl-3-(2′-chlor-3′,3′,3′-trifluorpropenyl)-cyclopropancarbonsäure die bei Raumtemperatur flüssig bleibt. Kp. 90—93°C/0,6 mm (0,8 mbar).

Beispiel 19

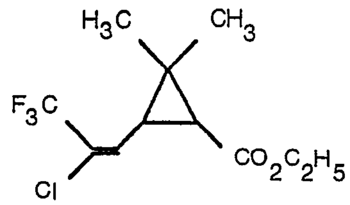

Kp: 103°C/16 mm (21,3 mbar)
$n_D^{20}$: 1.4366 und

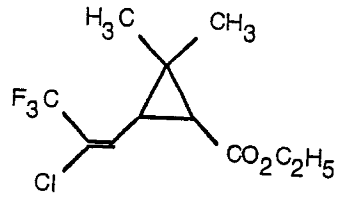

Kp: 102°C/16 mm (21,3 mbar)
$n_D^{20}$: 1.4356
sowie ein drittes Isomeres, vermutlich

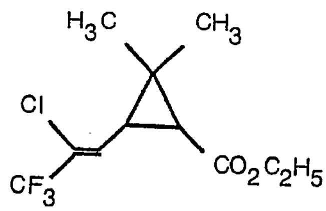

Kp: 105°C/16 mm (21,3 mbar)
$n_D^{20}$: 1.4364
wurden durch Destillation an einer Ringspaltkolonne rein aus dem Isomerengemisch, das bei der Herstellung des 2,2-Dimethyl-3-(2′-chlor-3′,3′,3′-trifluormethyl)-cyclopropancarbonsäureäthylesters, anfällt, isoliert.

Beispiel 20

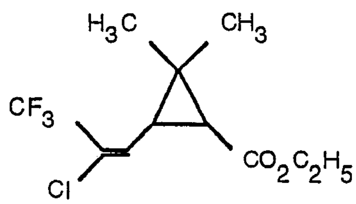

Zu einer Lösung von 20,7 g Natrium in 300 ml Äthanol werden innerhalb von 3 h unter Rühren bei Raumtemperatur 135 g 3,3-Dimethyl-4,6,6-trichlor-7,7,7-trifluorönanthsäuremethylester getropft. Man rührt 1 h bei Raumtemperatur nach und erhitzt dann 7 h auf 45°C. Nach dem Abkühlen filtriert man von abgeschiedenen Kochsalz ab, engt im Vakuum etwas ab, gibt auf 500 ml Wasser und stellt neutral. Durch Extrahieren mit Methylenchlorid und fraktionierte Destillation erhält man 90 g 2,2-Dimethyl-3-(2′-chlor-3′,3′,3′-trifluorpropenyl)-cyclopropencarbonsäureäthylester (Isomerengemisch) vom Kp: 55—57°C/0,2 mm (0,266 mbar).

Der Methylester wird mit Na-Methylat in Methanol erhalten.

Beispiel 21

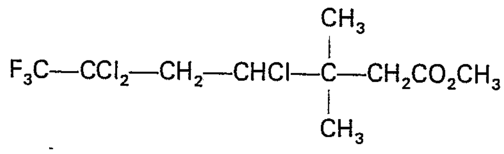

37,5 g 3,3-Dimethyl-4-pentensäuremethylester, 99,4 g 1,1,1-Trichlor-2,2,2-trifluoräthan, 22 g Acetonitril, 325 mg $FeCl_3.6H_2O_2$ 255 mg Benzoin und 200 mg Dimethylaminhydrochlorid werden in einem 250 ml VA-Autoklaven unter 6 bar Stickstoff 7 Stunden auf 100—120°C erhitzt und über eine 50 cm Vigreuxkolonne destilliert. Man erhält 70 g 3,3-Dimethyl-4,6,6-trichlor-7,7,7-trifluorönanthsäuremethylester vom Kp: 88—92°C/0,15 Torr (0,2 mbar).

Analog wurden erhalten:

| Beispiel | Verbindung | Kp. (°C/mm) |
|---|---|---|
| 22 | 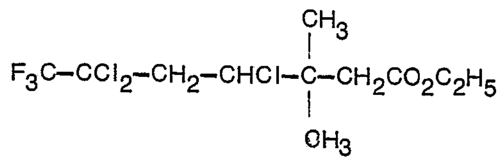 | 98 – 102/0,2 (0,266 mbar) |
| 23 | 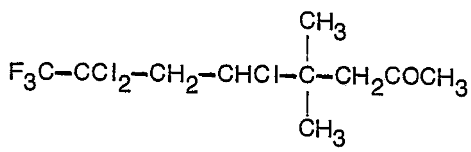 | 82 – 88/0,2 (0,266 mbar) |
| 24 | 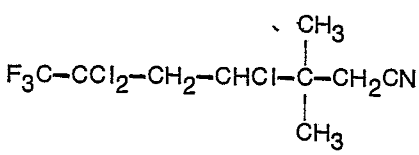 | 104 – 110/0,25 (0,33 mbar) |
| 25 | 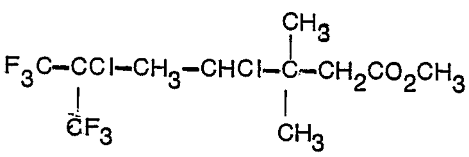 | 76 – 83/0,2 (0,266 mbar) |

**Patentansprüche für die Vertragsstaaten: DE BE CH FR GB NL SE**

1. Fluoralkenylsubstituierte Cyclopropancarbonsäurederivate der Formel I

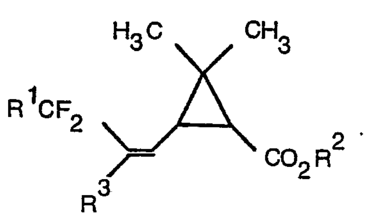

(I)

in welcher

$R^1$ für Fluor oder $CF_3$ steht,

$R^3$ für Fluor, Chlor, Brom oder den Rest $R^1CF_2$-steht und

$R^2$ für den Rest Formel steht:

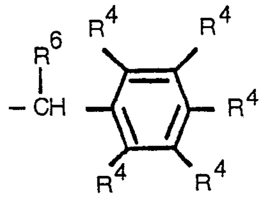

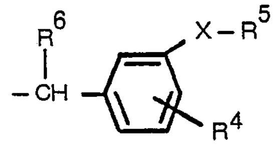

wobei

X für —$CH_2$—, —O—, oder —S— steht,
$R^4$ für Wasserstoff, Halogen oder $CF_3$ steht,
$R^5$ für —$CH_2$—C≡CH, —$CHF_2$, —$CF_3$, —CH=$CCl_2$ oder einen gegebenenfalls durch ein oder mehrere Halogenatome, Methyloder Methoxygruppen substituierten Phenylrest steht
$R^6$ für H, oder CN steht,
ausgenommen die Verbindung 2,2-Dimethyl-3-(2′-chlor-3′,3′,3′trifluorpropenyl)-cyclopropancarbonsäure-(3′-phenoxy)-benzylester und (3′-phenoxy)-$\alpha$-CN-benzylester.

2. Verfahren zur Herstellung der fluoralkenylsubstituierten Cyclopropancarbonsäurederivate der Formel I

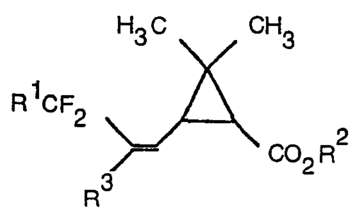

(I)

in welcher
$R^1$ für Fluor oder $CF_3$ steht,
$R^3$ für Fluor, Chlor oder den Rest $R^1CF_2$- steht und
$R^2$ für den Rest der Formel steht

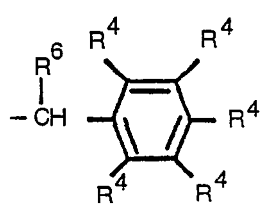

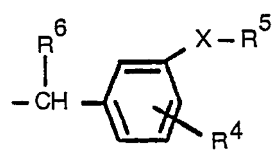

worin
X für —$CH_2$—, —O—, oder —S— steht,
$R^4$ für Wasserstoff, Halogen oder $CF_3$ steht,
$R^5$ für —$CH_2$—C≡CH, —$CHF_2$, —$CF_3$, —CH=$CCl_2$ oder einen gegebenenfalls durch ein oder mehrere Halogenatome, Methyl- oder Methoxygruppen substituierten Phenylrest steht
$R^6$ für H, oder CN steht, ausgenommen die Verbindung 2,2 - Dimethyl - 3 - (2′ - chlor - 3′,3′,3′ - trifluoropropenyl) - cyclopropancarbonsäure - (3′ - phenoxy) - benzylester und (3′-phenoxy) - $\alpha$ - CN - benzylester, dadurch gekennzeichnet, daß man eine Säure oder deren reaktionsfähiges Derivat der Formel II

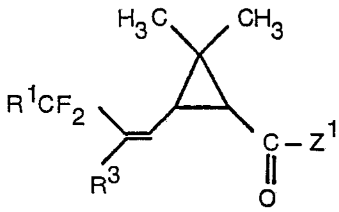

(II)

in welcher

$R^1$ und $R^3$ die oben angegebene Bedeutung haben und

$Z^1$ Halogen oder OH bedeutet, mit einen Alkohol oder dessen reaktionsfähigem Derivat der Formel III

$$R^2—Z^2 \quad (III)$$

in welcher

$R^2$ die oben angegebene Bedeutung hat und

$Z^2$ OH, Cl oder Br bedeutet,

gegebenenfalls in Gegenwart von Lösungsmitteln, Säureakzeptoren und/oder Phasentransferkatalysatoren, umsetzt.

3. Insektizide und/oder akarizide Mittel, gekennzeichnet durch einen Gehalt an mindestens einem fluoralkenylsubstituierten Cyclopropancarbonsäureester der Formel (I).

4. Verwendung von fluoralkenylsubstituierten Cyclopropancarbonsäureestern der Formel (I) zur Bekämpfung von Insekten und/oder Spinnentieren.

5. Verbindungen der Formel (II)

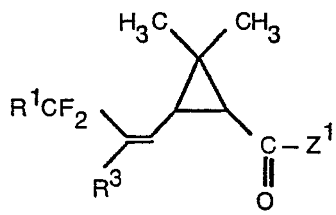

(II)

in welcher $R^1$, $R^3$ und $Z^1$ die in Anspruch 2 angegebene Bedeutung haben mit Ausnahme der 2,2-Dimethyl-3-(2′-chlor-3′,3′,3′-trifluorpropenyl)-cyclopropancarbonsäure und ihres Säurechlorids.

6. Verbindungen der Formel (IV)

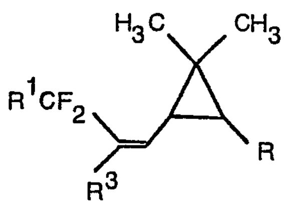

(IV)

in welcher R $—COCH_3$, $—COOC_2—C_4$-Alkyl oder —CN bedeuten und $R^1$ und $R^3$ die im Anspruch 1 angegebene Bedeutung haben mit Ausnahme des 2,2-Dimethyl-3-(2′-chlor-3′,3′,3′-trifluorpropenyl)-cyclopropancarbonsäureethylesters.

**Patentansprüche für die Vertragsstaaten: AT IT**

1. Fluoralkenylsubstituierte Cyclopropancarbonsäurederivate der Formel I

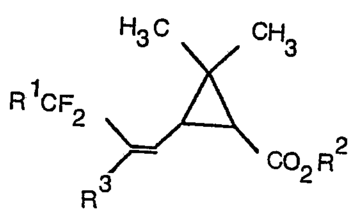

(I)

in welcher

$R^1$ für Fluor oder $CF_3$ steht,
$R^3$ für Fluor, Chlor, Brom oder den Rest $R^1CF_2$-steht und
$R^2$ für den Rest der Formel steht:

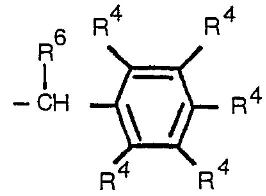

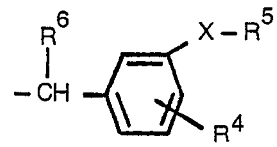

wobei

X für $—CH_2—$, —O—, oder —S— steht,
$R^4$ für Wasserstoff, Halogen oder $CF_3$ steht,
$R^5$ für $—CH_2—C{\equiv}CH$, $—CF_2$, $—CF_3$, $—CH{=}CCl_2$ oder einen gegebenenfalls durch ein oder mehrere Halogenatome, Methyloder Methoxygruppen substituierten Phenylrest steht,
$R^6$ für H oder CN steht.

2. Verfahren zur Herstellung der Fluoralkenylsubstituierten Cyclopropancarbonsäurederivate der Formel I

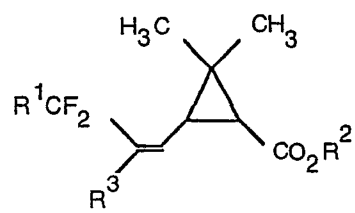

(I)

in welcher
$R^1$ für Fluor oder $CF_3$ steht,
$R^3$ für Fluor, Chlor, Brom oder den Rest $R^1CF_2$- steht und
$R^2$ für der Formel steht

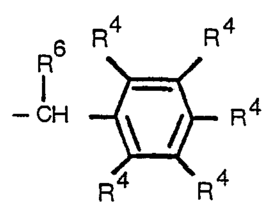

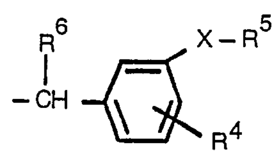

wobei
X für $—CH_2—$, —O—, oder —S— steht,
$R^4$ für Wasserstoff, Halogen oder $CF_3$ steht,
$R^5$ für $—CH_2—C{\equiv}CH$, $—CF_2$, $—CF_3$, $—CH{=}CCl_2$ oder einen gegebenenfalls durch ein oder mehrere Halogenatome, Methyl- oder Methoxygruppen substituierten Phenylrest steht
$R^6$ für H, oder CN steht, dadurch gekennzeichnet, daß man eine Säure oder deren reaktionsfähiges Derivat der Formel II

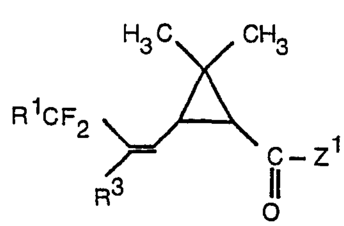

(II)

in welcher

$R^1$ und $R^3$ die oben angegebene Bedeutung haben und

$Z^1$ Halogen oder OH bedeutet, mit einem Alkohol oder dessen reaktionsfähigem Derivat der Formel III

$$R^2—Z^2 \qquad (III)$$

in welcher

$R^2$ die oben angegebene Bedeutung hat und

$Z^2$ OH, Cl oder Br bedeutet,

gegebenenfalls in Gegenwart von Lösungsmitteln, Säureakzeptoren und/oder Phasentransferkatalysatoren, umsetzt.

3. Insektizide und/oder akarizide Mittel, gekennzeichnet durch einen Gehalt an mindestens einem fluoralkenylsubstituierten Cyclopropancarbonsäureester der Formel (I).

4. Verwendung von fluoralkenylsubstituierten Cyclopropancarbonsäureestern der Formel (I) zur Bekämpfung von Insekten und/oder Spinnentieren.

5. Verbindungen der Formel II

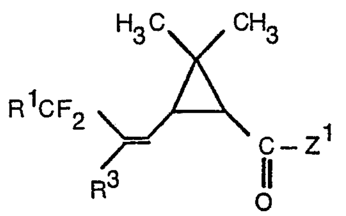

(II)

in welcher $R^1$, $R^3$ und $Z^1$ die im Anspruch 2 angegebene Bedeutung haben.

6. Verbindungen der Formel IV

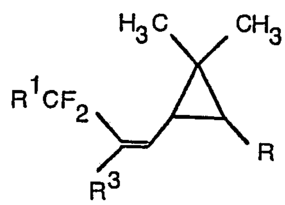

(IV)

in welcher R—$COCH_3$, —$COOC_1$—$C_4$-Alkyl oder CN bedeuten und $R^1$ und $R^3$ die in Anspruch 1 angegebene Bedeutung haben.

**Revendications pour les Etats contractants: BE CH DE FR GB NL SE**

1. Dérivés d'acides cyclopropanecarboxyliques à substituant fluoralcényle de formule I

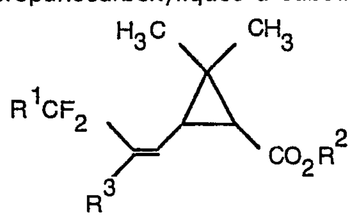

(I)

dans laquelle

$R^1$ représente le fluor ou le groupe $CF_3$

$R^3$ est le fluor, le chlore, le brome ou le reste $R^1CF_2$ et

$R^2$ est le reste de formule

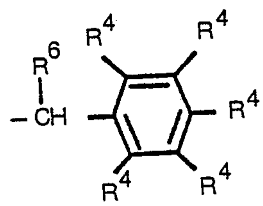

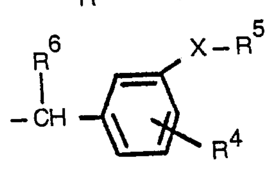

où

X représente —$CH_2$—, —O—, ou —S—,

$R^4$ est l'hydrogène, un halogène ou un groupe $CF_3$,

$R^5$ est un groupe —$CH_2$—C≡CH, —$CHF_2$, —$CF_3$, —CH=$CCl_2$ ou un reste phényle éventuellement substitué par un ou plusieurs atomes d'halogènes, groupes méthyle ou groupes méthoxy,

$R^6$ représente H ou le groupe CN,

à l'exception de l'ester de (3'-phénoxy)-benzyl et de l'ester de (3'-phénoxy)-$\alpha$-CN-benzyle de l'acide 2,2-diméthyl-3-(2'-chloro-3',3',3'-trifluoropropényl) cyclopropanecarboxylique.

2. Procédé de production de dérivés d'acides cyclopropanecarboxyliques à substituant fluoralcényle de formule I

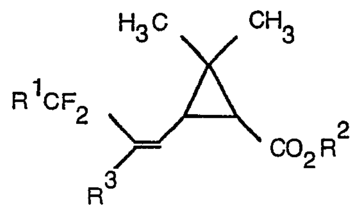

(I)

dans laquelle

$R^1$ représente le fluor ou le groupe $CF_3$,

$R^3$ est le fluor, le chlore ou le reste $R^1CF_2$— et

$R^2$ est le reste de formule

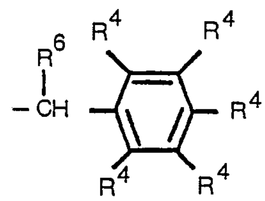

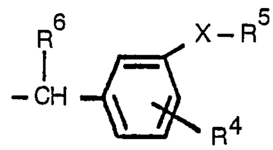

dans laquelle

X représente —$CH_2$—, —O—, ou —S—,

$R^4$ est l'hydrogène, un halogène ou le groupe $CF_3$,

$R^5$ est le groupe —$CH_2$—C≡CH, —$CHF_2$, —$CF_3$, —CH=$CCl_2$ ou un reste phényle éventuellement substitué par un ou plusieurs atomes d'halogènes, groupes méthyle ou groupes méthoxy.

$R^6$ représente H ou le groupe CN,

à l'exception de l'ester de (3'-phénoxy)benzyle et de l'ester de (3'-phénoxy)-$\alpha$-CN-benzyle de l'acide 2,2-diméthyl-3-(2'-chloro-3',3',3'-trifluoropropényl)-cyclopropanecarboxylique, caractérisé en ce qu'on fait réagir un acide ou son dérivé réactif de formule II

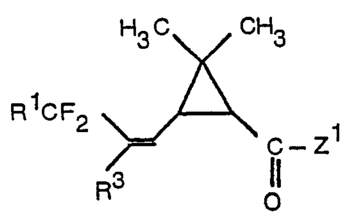

(II)

dans laquelle

$R^1$ et $R^3$ ont la définition indiquée ci-dessus et

$Z^1$ est un halogène ou le groupe OH,

avec un alcool ou son dérivé réactif de formule III

$$R^2—Z^2 \qquad (III)$$

dans laquelle

$R^2$ a la définition indiquée ci-dessus et

$Z^2$ représente OH, Cl ou Br,

éventuellement en présence de solvants, d'accepteurs d'acides et/ou de catalyseurs de transfert de phase.

3. Compositions insecticides et/ou acaricides, caractérisées par une teneur en au moins un ester d'acide cyclopropanecarboxylique à substituant fluoralcényle de formule (I).

4. Utilisation d'esters d'acides cyclopropanecarboxyliques à substituant fluoralcényle de formule (I) pour la lutte contre des insectes et/ou des acariens.

5. Composés de formule (II)

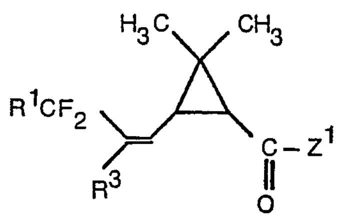

(II)

dans laquelle $R^1$, $R^3$ et $Z^1$ ont la définition donnée dans la revendication 2, excepté l'acide 2,2-diméthyl-3-(2'-chloro-3',3',3'-trifluoropropényl)-cyclopropanecarboxylique et son chlorure d'acide.

6. Composés de formule (IV)

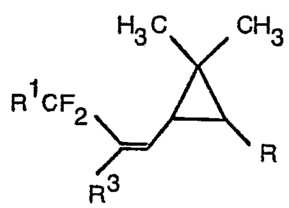

(IV)

dans laquelle R représente —$COCH_3$, —COO— (alkyle en $C_1$ à $C_4$) ou —CN et $R^1$ et $R^3$ ont la définition indiquée dans la revendication 1, à l'exception de l'ester éthylique de l'acide 2,2-diméthyl-3-(2'-chloro-3',3',3'-trifluoropropényl)-cyclopropanecarboxylique.

**Revendications pour les Etats contractants: AT IT**

1. Dérivés d'acides cyclopropanecarboxyliques à substituant fluoralcényle de formule I

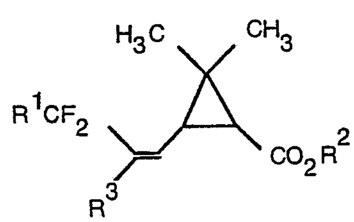

(I)

dans laquelle

$R^1$ représente le fluor ou le groupe $CF_3$

$R^3$ est le fluor, le chlore, le brome ou le reste $R^1CF_2$ et

$R^2$ est le reste de formule

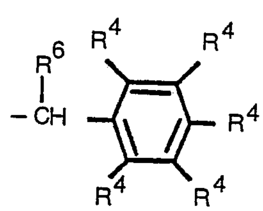

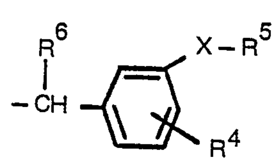

où

X représente —$CH_2$—, —O—, ou —S—,

$R^4$ est l'hydrogène, un halogène ou un groupe $CF_3$,

$R^5$ est un groupe $-CH_2-C\equiv CH$, $-CF_2$, $-CF_3$, $-CH=CCl_2$ ou un reste phényle éventuellement substitué par un ou plusieurs atomes d'halogènes, groupes méthyle ou groupes méthoxy,

$R^6$ représente H ou le groupe CN,

2. Procédé de production de dérivés d'acides cyclopropanecarboxyliques à substituant fluoralcényle de formule I

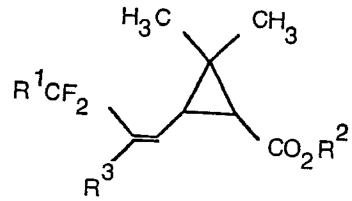

(I)

dans laquelle

$R^1$ représente le fluor ou le groupe $CF_3$,

$R^3$ est le fluor, le chlore, le brome ou le reste $R^1CF_2-$ et

$R^2$ est le reste de formule

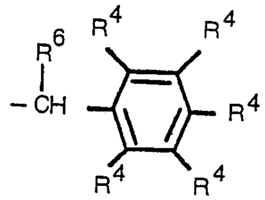

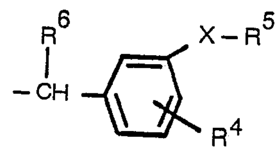

dans laquelle

X représente $-CH_2-$, $-O-$, ou $-S-$,

$R^4$ est l'hydrogène, un halogène ou le groupe $CF_3$,

$R^5$ est le groupe $-CH_2-C\equiv CH$, $-CF_2$, $-CF_3$, $-CH=CCl_2$ ou un reste phényle éventuellement substitué par un ou plusieurs atomes d'halogènes, groupes méthyle ou groupes méthoxy.

$R^6$ représente H ou le groupe CN, caractérisé en ce qu'on fait réagir un acide ou son dérivé réactif de formule II

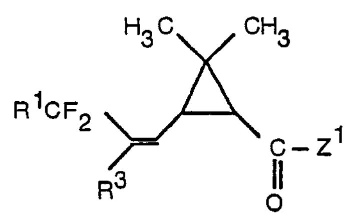

(II)

dans laquelle

$R^1$ et $R^3$ ont la définition indiquée ci-dessus et

$Z^1$ est un halogène ou le groupe OH,

avec un alcool ou son dérivé réactif de formule III

$$R^2-Z^2 \qquad (III)$$

dans laquelle

$R^2$ a la définition indiquée ci-dessus et

$Z^2$ représente OH, Cl ou Br,

éventuellement en présence de solvants, d'accepteurs d'acides et/ou de catalyseurs de transfert de phase.

3. Compositions insecticides et/ou acaricides, caractérisées par une teneur en au moins un ester d'acide cyclopropanecarboxylique à substituant fluoralcényle de formule (I).

4. Utilisation d'esters d'acides cyclopropanecarboxyliques à substituant fluoralcényle de formule (I) pour la lutte contre des insectes et/ou des acariens.

5. Composés de formule (II)

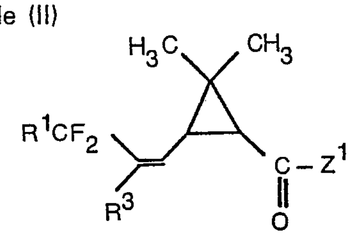

(II)

dans laquelle $R^1$, $R^3$ et $Z^1$ ont la définition indiquée dans la revendication 2.

6. Composés de formule (IV)

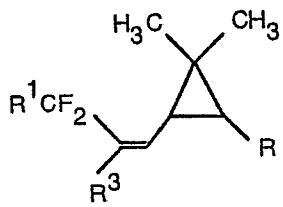

(IV)

dans laquelle R représente —$COCH_3$, —COO(alkyle en $C_1$ à $C_4$) ou —CN et $R^1$ et $R^3$ ont la définition indiquée dans la revendication 1.

**Claims for the Contracting States: BE CH DE FR GB NL SE**

1. Fluoroalkenyl-substituted cyclopropanecarboxylic acid derivatives of the formula I

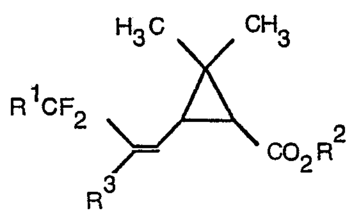

(I)

in which

$R^1$ represents fluorine or $CF_3$,
$R^3$ represents fluorine, chlorine, bromine or the radical $R^1CF_2$- and
$R^2$ represents the radical of the formula:

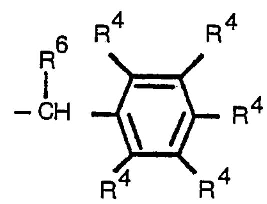

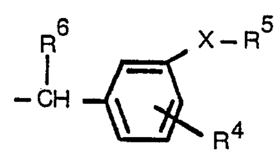

wherein

$X_4$ represents —$CH_2$, —O—, or —S—,
$R^4$ represents hydrogen, halogen or $CF_3$,
$R^5$ represents —$CH_2$—C≡CH, —$CHF_2$, —$CF_3$, —CH=$CCl_2$ or a phenyl radical which is optionally substituted by one or more halogen atoms, methyl or methoxy groups and
$R^6$ represents H, or CN,

with the exception of the compounds 2,2-dimethyl-3-(2′-chloro-3′,3′,3′-trifluoropropenyl)-cyclopropanecarboxylic acid (3′-phenoxy)-benzyl ester and 2,2-dimethyl-3-(2′-chloro-3′,3′,3′-trifluoropropenyl)-cyclopropanecarboxylic acid (3′-phenoxy-α-CN-benzyl ester.

2. Process for the preparation of the fluoroalkenyl-substituted cyclopropanecarboxylic acid derivatives of the formula I

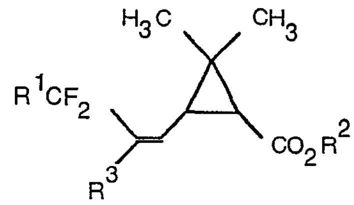

(I)

in which

$R^1$ represents fluorine or $CF_3$,

$R^3$ represents fluorine, chlorine or the radical $R^1CF_2$- and

$R^2$ represents the radical of the formula

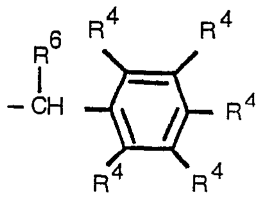

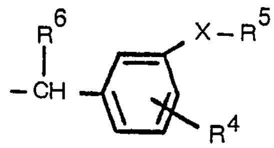

wherein

X represents $-CH_2$, —O—, or —S—,

$R^4$ represents hydrogen, halogen or $CF_3$,

$R^5$ represents $-CH_2-C\equiv CH$, $-CHF_2$, $-CF_3$, $-CH=CCl_2$ or a phenyl radical which is optionally substituted by one or more halogen atoms, methyl or methoxy groups and

$R^6$ represents H, or CN,

with the exception of the compounds 2,2-dimethyl-3-(2′-chloro-3′,3′,3′-trifluoropropenyl)-cyclopropanecarboxylic acid (3′-phenoxy)-benzyl ester and 2,2-dimethyl-3-(2′-chloro-3′,3′,3′,-trifluoropropenyl)-cyclopropanecarboxylic acid (3′-phenoxy-$\alpha$-CN-benzyl ester, characterized in that an acid or reactive derivative thereof, of the formula II

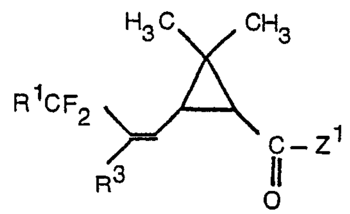

(II)

in which

$R^1$ and $R^3$ have the meaning indicated above and

$Z^1$ denotes halogen or OH,

is reacted with an alcohol or reactive derivative thereof of the formula III

$$R^2—Z^2 \qquad \text{(III)}$$

in which

$R^2$ has the meaning indicated above and

$Z^2$ denotes OH, Cl or Br,

if appropriate in the presence of solvents, acid acceptors and/or phase transfer catalysts.

3. Insecticidal and/or acaricidal agents, characterised in that they contain at least one fluoroalkenyl-substituted cyclopropanecarboxylic acid ester of the formula (I).

4. Use of fluoroalkenyl-substituted cyclopropanecarboxylic acid esters of the formula (I) for combating insects and/or arachnidae.

5. Compounds of the formula (II)

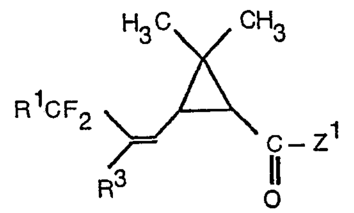

(II)

in which

$R^1$, $R^3$ and $Z^1$ have the meaning indicated in Claim 2 with the exception of 2,2-dimethyl-3-(2′-chloro-3′,3′,3′,-trifluoropropenyl)-cyclopropanecarboxylic acid and the acid chloride thereof.

6. Compounds of the formula (IV)

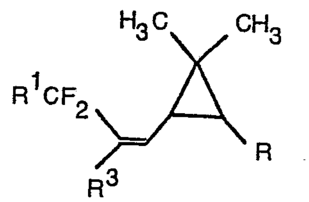

(IV)

in which

R denotes $—COCH_3$, $—COOC_1—C_4$-alkyl or —CN and

$R^1$ and $R^3$ have the meaning indicated in Claim 1, with the exception of 2,2-dimethyl-3-(2′-chloro-3′,3′,3′-trifluoropropenyl)-cyclopropanecarboxylic acid ethyl ester.

**Claims for the Contracting States: AT IT**

1. Fluoroalkenyl-substituted cyclopropanecarboxylic acid derivatives of the formula I

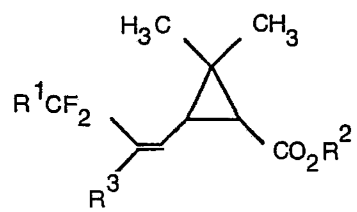

(I)

in which

$R^1$ represents fluorine or $CF_3$,

$R^3$ represents fluorine, chlorine, bromine or the radical $R^1CF_2$- and

$R^2$ represents the radical of the formula:

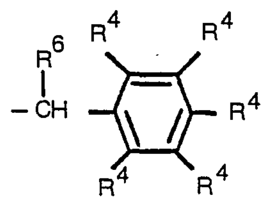

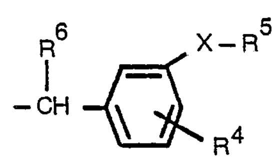

wherein

X represents $—CH_2$, —O—, or —S—,

$R^4$ represents hydrogen, halogen or $CF_3$,

$R^5$ represents $—CH_2—C{\equiv}CH$, $—CF_2$, $—CF_3$, $—CH{=}CCl_2$ or a phenyl radical which is optionally substituted by one or more halogen atoms, methyl or methoxy groups and

$R^6$ represents H, or CN.

2. Process for the preparation of the fluoroalkenyl-substituted cyclopropanecarboxylic acid derivatives of the formula I

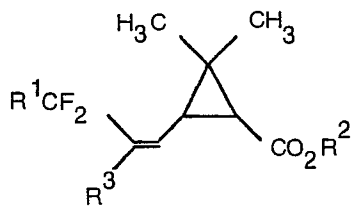

(I)

in which

$R^1$ represents fluorine or $CF_3$,

$R^3$ represents fluorine, chlorine bromine or the radical $R^1CF_2$- and

$R^2$ represents the radical of the formula

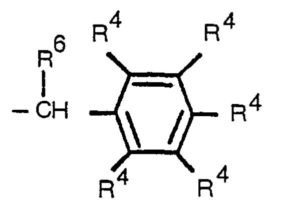

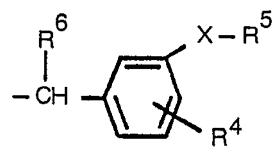

wherein

X represents $—CH_2$, —O—, or —S—,

$R^4$ represents hydrogen, halogen or $CF_3$,

$R^5$ represents $—CH_2—C{\equiv}CH$, $—CF_2$, $—CF_3$, $—CH{=}CCl_2$ or a phenyl radical which is optionally substituted by one or more halogen atoms, methyl or methoxy groups and

$R^6$ represents H, or CN,

characterised in that an acid or reactive derivative thereof, of the formula II

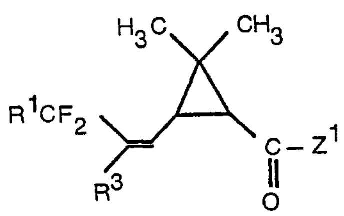

(II)

in which

$R^1$ and $R^3$ have the meaning indicated above and

$Z^1$ denotes halogen or OH,

is reacted with an alcohol or reactive derivative thereof of the formula III

$$R^2—Z^2 \qquad \text{(III)}$$

in which

$R^2$ has the meaning indicated above and

$Z^2$ denotes OH, Cl or Br,

if appropriate in the presence of solvents, acid acceptors and/or phase transfer catalysts.

3. Insecticidal and/or acaricidal agents, characterised in that they contain at least one fluoroalkenyl-substituted cyclopropanecarboxylic acid ester of the formula (I).

4. Use of fluoroalkenyl-substituted cyclopropanecarboxylic acid esters of the formula (I) for combating insects and/or arachnidae.

5. Compounds of the formula (II)

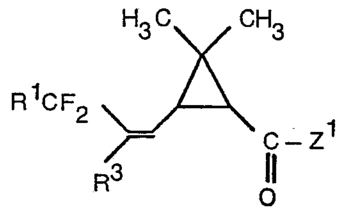 (II)

in which

$R^1$, $R^3$ and $Z^1$ have the meaning indicated in Claim 2.

6. Compounds of the formula (IV)

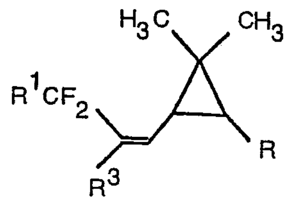 (IV)

in which

R denotes $-COCH_3$, $-COOC_1-C_4$-alkyl or CN and

$R^1$ and $R^3$ have the meaning indicated in Claim 1.